# EUROPEAN PATENT APPLICATION

(11) **EP 4 691 434 A1**
(43) Date of publication of application: **11.02.2026**
(21) Application number: 23930766.3
(22) Date of filing: 02.10.2023
(51) Int. Cl.: A61F 13/15

(54) **INDIVIDUALLY WRAPPED ABSORBENT ARTICLE**

(30) Priority: 30.03.2023 JP 2023055945
(71) Applicant: DAIO PAPER CORPORATION, Shikokuchuo-shi Ehime 799-0492 (JP)
(72) Inventor: FUKUDA, Juri, Sakura-shi, Tochigi 329-1411 (JP); KURAMOCHI, Mihoko, Sakura-shi, Tochigi 329-1411 (JP); ITO, Rina, Sakura-shi, Tochigi 329-1411 (JP); IWABUCHI, Haruka, Sakura-shi, Tochigi 329-1411 (JP); TAKAHASHI, Hiromi, Sakura-shi, Tochigi 329-1411 (JP)
(74) Representative: Epping - Hermann - Fischer
(86) International application number: PCT/JP2023/035899
(87) International publication number: WO 2024/202135

(57) **Abstract**

[Object] To avoid reduction in production efficiency when multiple individually-wrapped absorbent articles are stacked and encased in a package.

[Solution] An individually-wrapped absorbent article includes a wrapping sheet including paper, and an absorbent article individually wrapped with the wrapping sheet. Antislip is provided to a surface of the individually-wrapped absorbent article to be in contact with another individually-wrapped absorbent article when multiple individually-wrapped absorbent articles are stacked.

## Description

### TECHNICAL FIELD

The present invention relates to individually-wrapped absorbent articles.

### BACKGROUND ART

Typically, absorbent articles, such as sanitary napkins, pantyliners, incontinence pads, and the like, are provided in a state in which an absorbent article is individually encased in a package.

Patent Document 1 discloses a technology in which a mat layer having a relief pattern serving as an outermost layer is provided to a surface of a package, which encases an absorbent article, so that a friction force of the surface of the package is increased, thereby preventing load shifting during stacking of packages for display.

### RELATED-ART DOCUMENTS

### PATENT DOCUMENTS

Patent Document 1: Japanese Unexamined Patent Application Publication No. 2020-196541

### SUMMARY OF THE INVENTION

### PROBLEM TO BE SOLVED BY THE INVENTION

From reasons of hygiene during storage, convenience when carrying, and the like, absorbent articles, such as sanitary napkins, pantyliners, incontinence pads, and the like, are provided in packages as individually-wrapped absorbent articles in each of which an absorbent article is individually wrapped with a wrapping sheet. Since the wrapping sheet individually wraps an absorbent article, use of the wrapping sheet increases an amount of waste generated after use, compared with a case where a package is used. In recent years, use of a paper material to constitute a wrapping sheet has been promoted, instead of a non-woven fabric or a plastic film of the related art, in view of environmental issues.

The above-described individually-wrapped absorbent article is encased in a package in a state in which multiple individually-wrapped absorbent articles are stacked. Particularly in the case where paper having high surface smoothness is used as a wrapping sheet, an individually-wrapped absorbent articles are likely to slip against another individually-wrapped absorbent article when multiple individually-wrapped absorbent articles are stacked and encased in a package, thereby lowering production efficiency.

The present invention has been made in view of the above-described problems existing in the related art, and an object of the present invention is to provide an individually-wrapped absorbent article that can avoid reduction in production efficiency when multiple individually-wrapped absorbent articles are stacked and encased in a package.

### SOLUTION TO THE PROBLEM

In order to achieve the above object, the present invention is directed to an individually-wrapped absorbent article that includes a wrapping sheet including paper, and an absorbent article individually wrapped with the wrapping sheet. Antislip is provided to a surface of the individually-wrapped absorbent article to be in contact with another individually-wrapped absorbent article when multiple individually-wrapped absorbent articles are stacked.

In the present invention as configured above, antislip is provided to the surface of the individually-wrapped absorbent article to be in contact with another individually-wrapped absorbent article when multiple individually-wrapped absorbents are stacked, and therefore the individually-wrapped absorbent article is less likely to slip against another individually-wrapped absorbent article when multiple individually-wrapped absorbent articles are stacked and encased in a package, so that reduction in production efficiency can be avoided.

Moreover, the individually-wrapped absorbent article may be configured such that an antislip agent is applied to an entire area of an exposed surface of the wrapping sheet, which is exposed when the wrapping sheet individually wraps the absorbent article.

With the individually-wrapped absorbent article as configured above, the individually-wrapped absorbent article is less likely to slip against another individually-wrapped absorbent article regardless of a manner in which the multiple individually-wrapped absorbent articles are stacked.

Further, the individually-wrapped absorbent article may be configured such that within the exposed surface of the wrapping sheet, which is exposed when the wrapping sheet individually wraps the absorbent article, the antislip agent is applied to at least part of a region of the exposed surface in which a number of portions of the wrapping sheet overlap is the largest when the wrapping sheet individually wraps the absorbent article.

In the individually-wrapped absorbent article as configured above, the antislip agent is applied to the region of the individually-wrapped absorbent article that is most likely to be in contact with another individually-wrapped absorbent article when multiple individually-wrapped absorbent articles are stacked. Thus, the individually-wrapped absorbent article is less likely to slip against another individually-wrapped absorbent article when the multiple individually-wrapped absorbent articles are stacked and encased in a package. Since the individually-wrapped absorbent article is configured such that the antislip agent is applied to only part of the wrapping sheet, increase in the cost can be minimized.

Further, the individually-wrapped absorbent article is configured such that the individually-wrapped absorbent article further includes a fastening tape. The wrapping sheet includes a first region including one end in a longitudinal direction of the wrapping sheet, and a second region including the other end in the longitudinal direction. The wrapping sheet is folded so that the second region overlaps the first region, and the fastening tape is provided over the first region and the second region. When a machine MD direction of the wrapping sheet is a lateral direction orthogonal to the longitudinal direction, a length of the fastening tape in the longitudinal direction is 35% or greater relative to a length of the wrapping sheet in the longitudinal direction in a state in which the wrapping sheet is folded.

With the individually-wrapped absorbent article as configured above, the individually-wrapped absorbent article is less likely to slip against another individually-wrapped absorbent article owing to the fastening tape when the multiple individually-wrapped absorbent articles are stacked and encased in a package.

Further, the individually-wrapped absorbent article is configured such that the individually-wrapped absorbent article further includes a fastening tape. The wrapping sheet includes a first region including one end in a longitudinal direction of the wrapping sheet, and a second region including the other end in the longitudinal direction, the wrapping sheet is folded so that the second region overlaps the first region, and the fastening tape is provided over the first region and the second region. When a machine MD direction of the wrapping sheet is the longitudinal direction, a length of the fastening tape in a lateral direction orthogonal to the longitudinal direction is 15% or greater relative to a length of the wrapping sheet in the lateral direction.

With the individually-wrapped absorbent article as configured above, the individually-wrapped absorbent article is less likely to slip against another individually-wrapped absorbent article owing to the fastening tape when the multiple individually-wrapped absorbent articles are stacked and encased in a package.

Further, the individually-wrapped absorbent article may be configured such that a surface of the fastening tape has an antislip structure.

With the individually-wrapped absorbent article as configured above, the individually-wrapped absorbent article is less likely to slip against another individually-wrapped absorbent article owing to the fastening tape when the multiple individually-wrapped absorbent articles are stacked and encased in a package.

Further, the individually-wrapped absorbent article is configured such that an opposite surface of the wrapping sheet to an exposed surface of the wrapping sheet, which is exposed when the wrapping sheet individually wraps the absorbent article, has a pseudo-adhesive structure that releasably bonds the absorbent article to the wrapping sheet. An adhesive constituting the pseudo-adhesive structure seeps through the wrapping sheet to be present on the exposed surface of the wrapping sheet.

With the individually-wrapped absorbent article as configured above, the individually-wrapped absorbent article is less likely to slip against another individually-wrapped absorbent article owing to the pseudo-adhesive that releasably bonds the absorbent article to the wrapping sheet.

Further, the individually-wrapped absorbent article may be configured such that relief patterning is provided to an exposed surface of the wrapping sheet, which is exposed when the wrapping sheet individually wraps the absorbent article.

With the individually-wrapped absorbent article as configured above, the individually-wrapped absorbent article is less likely to slip against another individually-wrapped absorbent article owing to the relief patterning provided to the surface of the wrapping sheet, when the multiple individually-wrapped absorbent articles are stacked and encased in a package.

Further, the individually-wrapped absorbent article may be configured such that the relief patterning includes embossing.

With the individually-wrapped absorbent article as configured above, the individually-wrapped absorbent article is less likely to slip against another individually-wrapped absorbent article owing to the embossing provided to the surface of the wrapping sheet, when the multiple individually-wrapped absorbent articles are stacked and encased in a package.

Further, the individually-wrapped absorbent article may be configured such that the relief patterning includes application of an ink by printing.

With the individually-wrapped absorbent article as configured above, the individually-wrapped absorbent article is less likely to slip against another individually-wrapped absorbent article owing to the application of the ink to the surface of the wrapping sheet by printing, when the multiple individually-wrapped absorbent articles are stacked and encased in a package.

Further, the individually-wrapped absorbent article may be configured such that the relief patterning includes perforation of the wrapping sheet.

With the individually-wrapped absorbent article as configured above, the individually-wrapped absorbent article is less likely to slip against another individually-wrapped absorbent article owing to the perforation of the wrapping sheet, when the multiple individually-wrapped absorbent articles are stacked and encased in a package.

### EFFECTS OF THE INVENTION

According to the present invention, reduction in production efficiency can be avoided when multiple individually-wrapped absorbent articles are stacked and encased in a package.

### BRIEF DESCRIPTION OF THE DRAWINGS

[Fig. 1] Fig. 1 is a plan view illustrating one embodiment of the individually-wrapped absorbent article of the present invention.
[Fig. 2] Fig. 2 is a plan view of a developed state of the individually-wrapped absorbent article illustrated in Fig. 1.
[Fig. 3] Fig. 3 is a cross-sectional view taken along the line I-I illustrated in Fig. 1.
[Fig. 4] Fig. 4 is an enlarged view of the section II illustrated in Fig. 1.
[Fig. 5] Fig. 5 is a view illustrating a state in which individually-wrapped absorbent articles illustrated in Figs. 1 to 4 are encased in a package.
[Fig. 6] Fig. 6 is a view illustrating a first embodiment of antislip.
[Fig. 7] Fig. 7 is a view illustrating a second embodiment of the antislip.
[Fig. 8] Fig. 8 is a cross-sectional view of a state in which an absorbent article is individually wrapped with the wrapping sheet illustrated in Fig. 7.
[Fig. 9] Fig. 9 is a view illustrating a third embodiment of the antislip.
[Fig. 10] Fig. 10 is a view illustrating a fourth embodiment of the antislip.
[Fig. 11] Fig. 11 is a view illustrating a fifth embodiment of the antislip.
[Fig. 12] Fig. 12 is a cross-sectional view illustrating a stacked state of a release liner, a hot-melt adhesive, and a wrapping sheet, illustrated in Fig. 11.
[Fig. 13] Fig. 13 is a view illustrating a sixth embodiment of the antislip.
[Fig. 14] Fig. 14 is a view illustrating a seventh embodiment of the antislip.
[Fig. 15] Fig. 15 is a view illustrating an eighth embodiment of the antislip.
[Fig. 16] Fig. 16 is a view for explaining a state in which the wrapping sheet illustrated in Fig. 15 is folded, and resultant multiple individually-wrapped absorbent articles are stacked.
[Fig. 17] Fig. 17 is a view illustrating a ninth embodiment of the antislip.

### DESCRIPTION OF EMBODIMENTS

Embodiments of the present invention will be described with reference to drawings hereinafter. In the drawings, the same referential numeral is assigned to the same or corresponding constituent component unless otherwise stated, and redundant description may be omitted.

### <Basic configuration of individually-wrapped absorbent article>

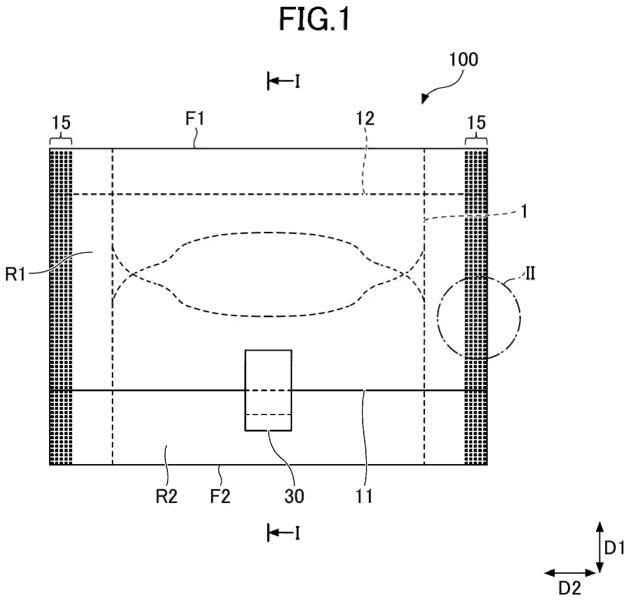

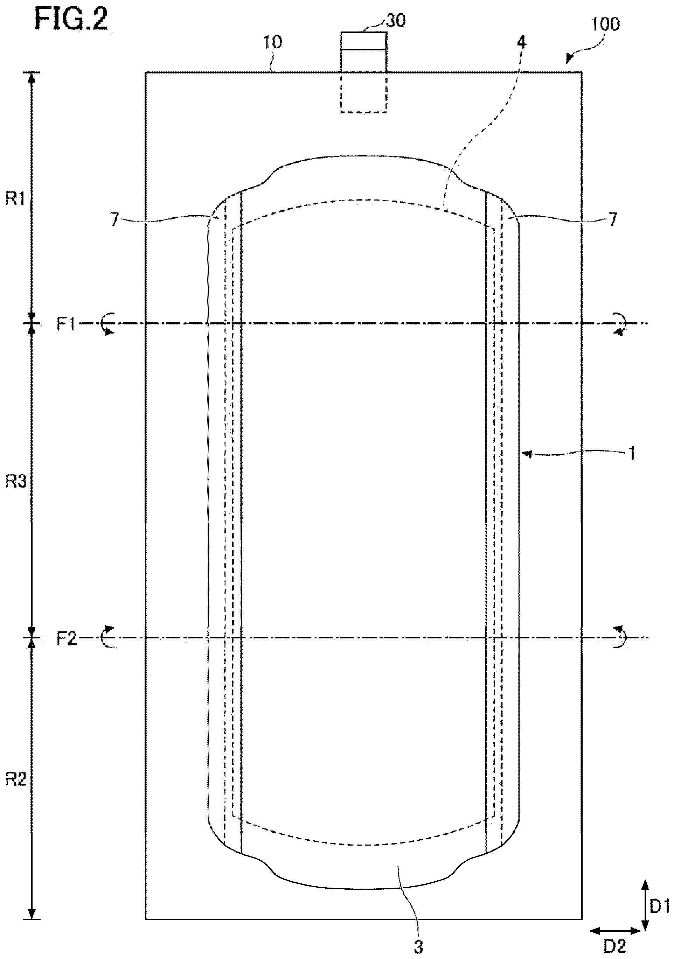

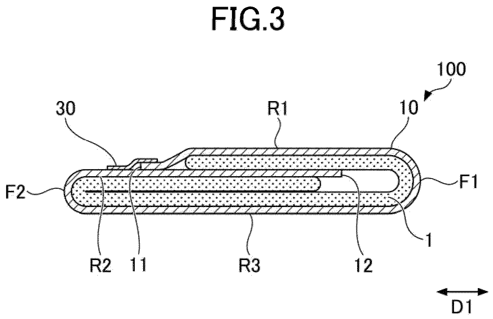

Fig. 1 is a plan view illustrating one embodiment of the individually-wrapped absorbent article of the present invention. Fig. 2 is a plan view of a developed state of the individually-wrapped absorbent article 100 illustrated in Fig. 1, and illustrates a view as viewed from the inner surface of the wrapping sheet 10 or the side of the absorbent article 1 facing the skin of a user. Moreover, Fig. 3 is a cross-sectional view taken along the line I-I illustrated in Fig. 1.

As illustrated in Figs. 1 to 3, the present embodiment is an individually-wrapped absorbent article 100 that includes a wrapping sheet 10, and an absorbent article 1 individually wrapped with the wrapping sheet 10. As illustrated in Fig. 2, the wrapping sheet 10 has, for example, a narrow and long shape in a developed state, and has a longitudinal direction (vertical direction) D1 and a lateral direction (horizontal direction) D2 orthogonal to the longitudinal direction. Moreover, the longitudinal direction D1 and the lateral direction D2 of the wrapping sheet 10 also correspond to a longitudinal direction and a lateral direction of the absorbent article 1, respectively.

### (Absorbent article)

The absorbent article 1 to be wrapped with the wrapping sheet 10 is an article having a shape that is suitable when the article is worn to face a discharge orifice of a body fluid (menstrual blood, vaginal discharge, urine, etc.), for example, a flat, narrow, and long shape. Specific examples of the absorbent article 1 includes sanitary napkins, pantyliners (vaginal discharge sheets), light incontinence pads, and the like.

The absorbent article 1 includes, for example, a fluid-permeable top sheet 3, a fluid-impermeable back sheet (not illustrated), and an absorber 4 disposed between the top sheet 3 and the back sheet, as illustrated in Fig. 2.

As the back sheet, a sheet having at least a water-shielding property, such as an olefin-based resin sheet or the like, may be used. The olefin-based resin is such as polyethylene, polypropylene, or the like. A laminate non-woven fabric, in which a non-woven fabric is laminated on a polyethylene sheet or the like, or a laminate sheet of non-woven fabrics, in which a waterproof film is inserted between the non-woven fabrics to substantially ensure a fluid impermeability, may be used as the back sheet. Further, a sheet having moisture permeability may be used as the back sheet.

As the top sheet 3, a porous or non-porous non-woven fabric, a porous plastic sheet, or the like is suitably used. As a fibrous material constituting the non-woven fabric, for example, one of, or two or more of synthetic fibers, such as olefin (e.g., polyethylene, polypropylene, etc.), polyester, polyamide, and the like, recycled fibers, such as rayon, cuprammonium rayon, and the like, blended fibers of the foregoing, and natural fibers, such as cotton and the like can be used.

The absorber 4 is not limited as long as the absorber 4 is a material that can absorb and retain a body fluid. The absorber 4 preferably includes cotton pulp and a water-absorbing polymer. As the water-absorbing polymer, a superabsorbent polymer (SAP) powder, a superabsorbent fiber (SAF), or a combination of the foregoing can be used. Examples of the pulp include chemical pulp obtained from wood, cellulose fibers, such as dissolving pulp, artificial cellulose fibers, such as rayon, acetate, and the like. The pulp may be hardwood pulp obtained from hardwood, softwood pulp obtained from softwood, or a pulp mixture of the foregoing. Moreover, the pulp may be recycled pulp that is recycled from used pulp.

A length of the absorber 4 may be from 0.5 mm to 25 mm. The absorber 4 may be configured such that a region corresponding to a body fluid discharge orifice (body-fluid-discharge-orifice corresponding region) or a region facing the intergluteal cleft behind the body-fluid-discharge-orifice corresponding region is bulged. The absorber 4 has a size and a shape that do not exceed the top sheet 3 and the back sheet. At the front and rear end edges of the absorber, the outer edges of the back sheet and the outer edges of the top sheet 3 are respectively joined by an adhesive such as a hot-melt adhesive or the like, or an adhesion method such as heat sealing or ultrasonic sealing.

As illustrated in Fig. 2, side sheets 7 may be respectively provided on the outer sides of the absorber 4 along the longitudinal direction D1 at the both edge portions of the absorber 4 in the lateral direction D2. As the side sheets 7, water-repellent non-woven fabrics or hydrophilic non-woven fabrics can be used.

Note that the absorbent article 1 is a so-called wingless type that does not have wings in the example illustrated in Figs. 1 to 3, but the absorbent article may be configured as an article having wings each extending to the side. The wings may be formed by joining the side sheet and the back sheet.

Moreover, an antislip adhesion portion may be provided on a back sheet side of the absorbent article 1 (the non-skin side, i.e., the side facing the underwear when worn). The antislip adhesion portion is provided for preventing the absorbent article 1 from being displaced from the underwear when the absorbent article 1 is attached to the underwear. The antislip adhesion portion is, for example, formed into multiple strips extending in the longitudinal direction D1 or the lateral direction D2.

An entire length of the absorbent article 1 can be from 140 mm to 430 mm, and a width of the absorbent article 1 (a width of a main body excluding wings if wings are provided) can be from 40 mm to 130 mm.

### (Wrapping sheet)

The wrapping sheet 10 of the present embodiment includes paper. Use of the wrapping sheet 10 made of paper can contribute to reduction in use of plastics, and to the achievement of sustainable development goals. Moreover, paper can provide unique textures, such as the appearance and feel that give gentle impressions natural materials have. In the present specification, the paper encompasses a thin flat sheet obtained by agglutinating plant fibers or other fibers with an agglutinating agent. In particular, the paper encompasses a sheet including plant fibers as a main raw material, for example, includes the plant fibers, particularly, cellulose fibers in an amount of 50% or greater, preferably 80% or greater, relative to all the fibers included in the sheet. The plant fibers (pulp) serving as a raw material of the paper may include wood pulp, non-wood pulp, and waste paper pulp, which may be mechanical pulp or chemical pulp. The pulp may be pulp recycled from constituent components of absorbent articles, wrapping sheets for absorbent articles, or both. Further, additives may be added to the paper. Specific examples of paper include various types of paper, such as Western paper, Japanese paper, processed paper, synthesis paper, and the like. Further, the paper may be paper used for other purposes in the related art, such as paper called newspaper, printing paper (including high quality paper), writing paper, drawing paper, packaging paper, tissue paper, hybrid paper, and the like. In the case of the tissue paper, the paper may be thin vellum paper, Indian paper, rice paper, glassine paper, tissue paper, toilet paper, filter paper, or the like.

In the present specification, the wrapping sheet made of paper encompasses a sheet mainly including the above paper. The wrapping sheet made of paper includes, not only a wrapping sheet composed only of paper, but also a laminate sheet in which paper and a sheet of a material other than paper are laminated. In the case where wrapping sheet 10 includes a sheet of a material other than paper, the material other than paper may be a resin film, a non-woven fabric, or the like. However, the wrapping sheet 10 is particularly preferably composed only of paper from the viewpoint of reduction in use of plastics, the viewpoint of natural texture unique to paper imparted to a product, or both.

The wrapping sheet 10 made of paper may be subjected to processing. The processing includes, for example, calendaring, water repellent processing, slitting, ply processing, printing, and the like. In the case where water repellent processing is performed on the wrapping sheet 10, for example, a water repellent agent, such as a silicone-based resin, a paraffin-based resin, a fluororesin, or the like, can be applied to at least an outer surface of the wrapping sheet 10 (the side of the wrapping sheet 10 to be exposed in the state in which the wrapping sheet 10 wraps the absorbent article 1) and an inner surface of the wrapping sheet 10 opposite to the outer surface. In the case where water repellent processing is performed on the inner surface of the wrapping sheet 10, an antislip adhesion portion disposed on a back surface of an absorbent article 1 becomes readily detachable from the wrapping sheet 10, and therefore a release liner can be omitted. Note that, when water repellent processing is performed on the wrapping sheet 10 in a production method where sealed portions are produced by pressure bonding with heated rolls in the present embodiment, a water-repellent agent may hinder formation of releasable sealed portions. Therefore, sealed portions 15 (described in detail later) are preferably not formed at both edge portions of an individually-wrapped absorbent article 100.

A size of the wrapping sheet 10 depends on a size or shape of an absorbent article 1 to be wrapped. For example, a length in the longitudinal direction D1 (may be merely referred to as a length) can be from 100 mm to 450 mm, and a length in the lateral direction D2 (may be merely referred to as a width) can be from 50 mm to 200 mm in the state in which the wrapping sheet 10 is completely extended (the unfolded state). In the illustrated example, the wrapping sheet 10 has a rectangular shape in a developed state, but may have, for example, a shape, such as an oblong shape.

### (Package configuration of individually-wrapped absorbent article)

As illustrated in Figs. 1 to 3, the absorbent article 1 is wrapped by being folded together with the wrapping sheet 10, thereby forming an individually-wrapped absorbent article 100. At the time of folding, first, the absorbent article 1 is placed on the inner surface of the wrapping sheet 10 in the manner such that the back sheet side of the absorbent article 1 faces the inner surface of the wrapping sheet 10, as illustrated in Fig. 2. Then, the wrapping sheet 10 and the absorbent article 1 are folded together in the longitudinal direction D1 at the first folding line F1 and the second folding line F2 along the width direction D2. More specifically, a first region R1 including one end 11 of the wrapping sheet 10 in the longitudinal direction D1 is folded over in the longitudinal direction D1 along the first folding line F1, and a second region R2 including the other end 12 of the wrapping sheet 10 in the longitudinal direction D1 is folded over along the second folding line F2. A region of the wrapping sheet 10 between the first region R1 and the second region R2 is a third region R3. In the illustrated example, the wrapping sheet 10 is folded in a manner such that the second region R2 is folded first, and then the first region R1 is folded to overlap the outer surface of the second region R2 (Figs. 1 and 2), but the order of folding the first region R1 and the second region R2 may be reversed. After the wrapping sheet 10 and the absorbent article 1 are together folded over in three (inward tri-fold) to wrap the absorbent article 1, both edge portions in the lateral direction D2 are sealed along the longitudinal direction D1, thereby forming sealed portions 15.

Such a wrapping form of three fold or greater can be formed relatively simply, and can wrap an absorbent article 1 hygienically. In addition, the absorbent article 1 can be easily taken out. Note that the folding form is not limited to three fold, and may be four fold or more, or may be two fold.

Further, the individually-wrapped absorbent article 100 may be provided with a fastening tape 30 at a center of the lateral direction D2 in the vicinity of the edge end of the first region R1 (one end 11 of the wrapping sheet 10). As illustrated in Fig. 1, the fastening tape 30 can be provided over the first region R1 and the second region R2 across one end 11 of the wrapping sheet 10. Since the fastening tape 30 is provided, a user can hold the fastening tape 30 to lift and pull the first region R1 at the time of opening the individually-wrapped absorbent article 100, and therefore the opening of the individually-wrapped absorbent article 100 is further facilitated. At the time of opening, the first region R1 is peeled from the second region R2 that is present below the first region R1.

### (Sealed portion)

As illustrated in Fig. 1, portions of the individually-wrapped absorbent article 100 where the absorbent article 1 is not present at both edges in the lateral direction D2, are respectively sealed, thereby forming sealed portions 15. In the present embodiment, the sealed portions 15 are formed by passing the wrapping sheet 10 through between a pair of heated rolls to pressure bond the above overlapped portions of the folded wrapping sheet 10 in a thickness direction.

As illustrated in Fig. 1, the sealed portions 15 are formed, for example, along an entire length of the individually-wrapped absorbent article 100 in the longitudinal direction D1. This is preferable from the viewpoint that erroneous entry of dust or dirt, or a finger or a small object from the end edges in the lateral direction can be prevented (sealability is assured).

A range of the lateral direction D2 in which each sealed portion 15 is provided is, for example, a range of 20 mm from the end edge in the lateral direction D2. The sealed portion 15 may be formed in the entire range, or may be formed in part of the above range, for example, at a position away from the end edge in the lateral direction D2. A length (width) of each sealed portion 15 itself in the lateral direction D2 is preferably from 3 mm to 15 mm. The inner edge of the sealed portion 15 in the lateral direction D2 may be linearly extended in the longitudinal direction D1, or extended in a curved manner, for example, in a wave-like manner.

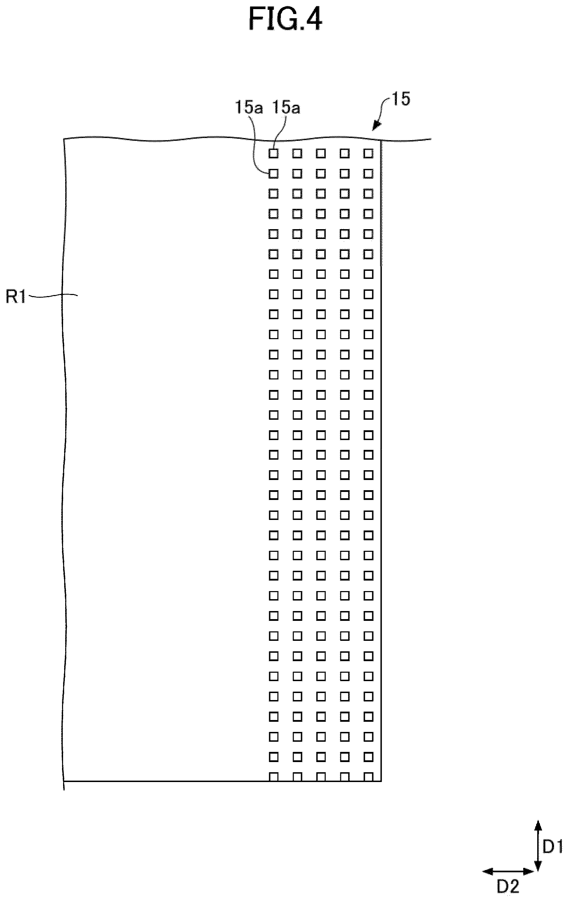

Fig. 4 is an enlarged view of the section II illustrated in Fig. 1, and illustrates a portion including the sealed portion 15.

As illustrated in Fig. 4, the sealed portion 15 includes multiple pressure-bonded portions (or bonded portions) 15a that are separated from one another in a plan view. Each pressure-bonded portion 15a is a portion in which overlapped portions of the folded wrapping sheet 10 are bonded by pressure bonding. In the illustrated example, a portion other than the pressure-bonded portions 15a is a non-bonded portion in which the overlapped portions of the folded wrapping sheet 10 are not bonded to each other. Since the pressure-bonded portions 15a are scattered apart from one another as described above, the bonded portions of the wrapping sheet can be easily peeled off at the time of opening, and openability can be improved, for example, as compared with a case where the portions of the wrapping sheets 10 are bonded over the entire surface of the sealed portion 15. Moreover, this can prevent the both edges of the individually-wrapped absorbent article 100 from becoming excessively hard and impairing the texture.

Note that a shape of each pressure-bonded portion 15a in a plan view is a square in the example illustrated in Fig. 4, but the shape is not limited to the illustrated shape and may be, for example, a quadrangle other than a square, such as a rectangle or a parallelogram, a polygon other than a quadrangle, a circle, an ellipse, a heart shape, a star shape, a drop shape, or the like. Moreover, a size of each pressure-bonded portion 15a is, for example, a square having a side of 0.25 mm to 2.5 mm or an equivalent size having the same area as the square.

The individually-wrapped absorbent article 100 is provided in a package in which the individually-wrapped absorbent article 100 is encased.

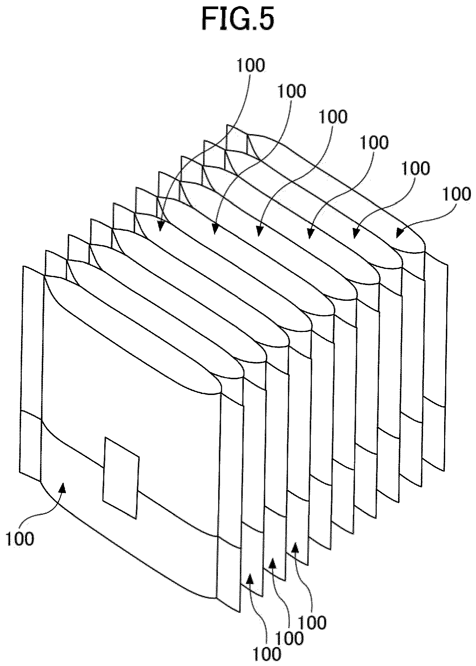

Fig. 5 is a view illustrating a state in which the individually-wrapped absorbent articles 100 each illustrated in Figs. 1 to 4 are encased in a package.

As illustrated in Fig. 5, the above-described individually-wrapped absorbent article 100 is encased in a package in a state in which the multiple individually-wrapped absorbent articles 100 are stacked. Particularly in the case where paper having high smoothness is used as the wrapping sheet 10, the individually-wrapped absorbent articles 100 easily slip against one another. Therefore, the stacked individually-wrapped absorbent articles 100 may come apart on the conveying line for conveying the individually-wrapped absorbent articles 100. The stacked individually-wrapped absorbent articles 100 are particularly likely to come apart in the machine MD direction. The above-described encasing of the individually-wrapped absorbent articles 100 in a package may be automatically performed on the conveying line. If the stacked individually-wrapped absorbent articles 100 come apart, therefore, the dispersed individually-wrapped absorbent articles 100 are manually encased in a package, which lowers production efficiency.

Therefore, for the individually-wrapped absorbent article 100 of the present embodiment, antislip is provided to a surface of the individually-wrapped absorbent article 100 to be in contact with another individually-wrapped absorbent article when the multiple individually-wrapped absorbent articles 100 are stacked. Embodiments of the antislip will be described hereinafter.

### <Antislip>

### (First embodiment)

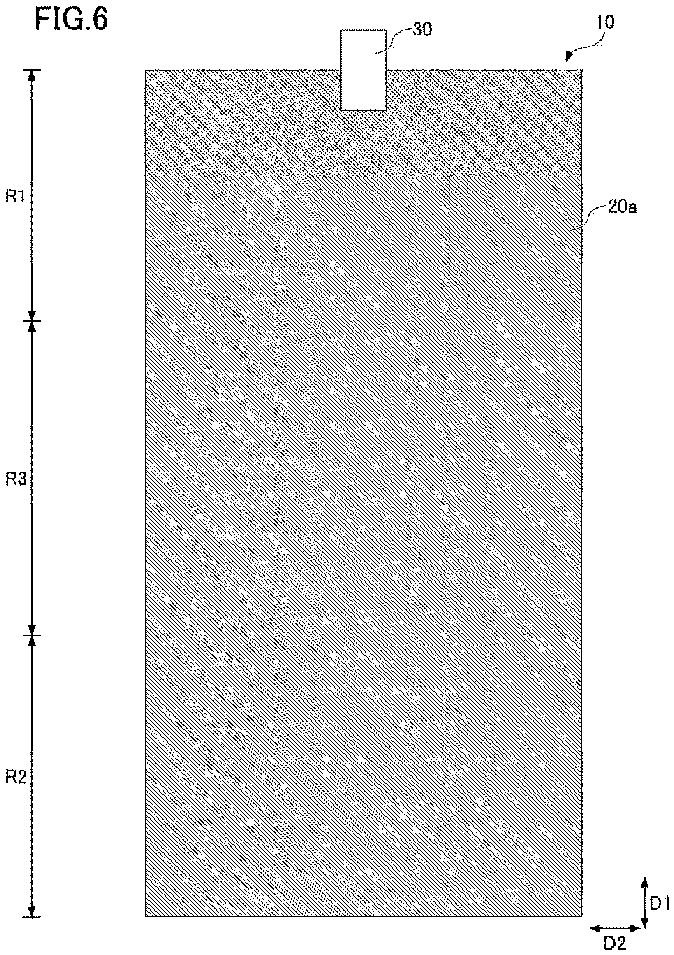

Fig. 6 is a view illustrating a first embodiment of the antislip, and is a view as viewed from an outer surface of the wrapping sheet 10.

As illustrated in Fig. 6, in the present embodiment, an antislip varnish 20a is applied to the entire area of the outer surface of the wrapping sheet 10. The outer surface is an exposed surface of the wrapping sheet 10, which is exposed when the wrapping sheet 10 individually wraps the absorbent article 1.

In the individually-wrapped absorbent article having the above configuration, the surface of the individually-wrapped absorbent article 100 is less slippery owing to the antislip varnish 20a applied to the wrapping sheet 10. In the case where multiple individually-wrapped absorbent articles are stacked as illustrated in Fig. 5, the individually-wrapped absorbent articles are therefore less likely to come apart. Thus, reduction in product efficiency can be avoided when multiple individually-wrapped absorbent articles 100 are stacked and encased in a package.

Since the antislip varnish 20a is applied to the entire area of the outer surface of the wrapping sheet 10, the individually-wrapped absorbent article 100 is less likely to slip against another individually-wrapped absorbent article 100 regardless of a manner in which the individually-wrapped absorbent articles 100 are stacked.

The antislip agent, which is applied to the exposed surface of the wrapping sheet 10 to make the surface of the wrapping sheet 10 less slippery, is not limited to the antislip varnish. For example, the antislip agent may be any material that causes the surface of the wrapping sheet 10 to be less slippery when the material is applied to the surface of the wrapping sheet 10, such as mat varnish. Specifically, a pressure-sensitive adhesive or a material causing rubber friction can be used. Further, when a silica-containing resin is used as the antislip agent, fine projected-portions can be formed on the surface of the wrapping sheet 10 so that the surface of the wrapping sheet 10 becomes less slippery due to friction.

### (Second embodiment)

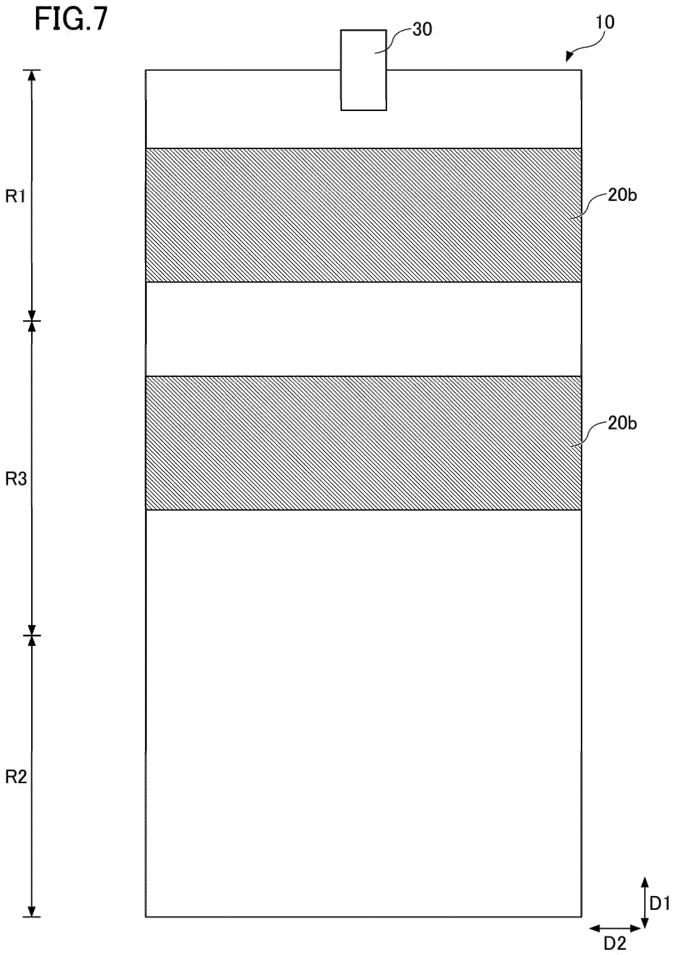

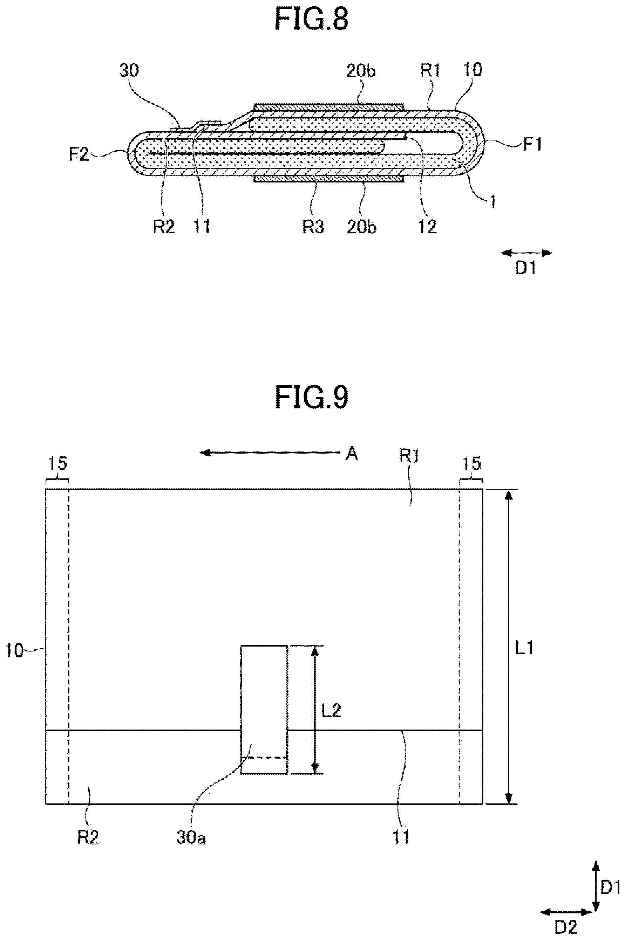

Fig. 7 is a view illustrating a second embodiment of the antislip, and is a view as viewed from the outer surface of the wrapping sheet 10. Fig. 8 is a cross-sectional view illustrating a state in which the wrapping sheet 10 illustrated in Fig. 7 individually wraps the absorbent article 1, and illustrates a cross-section taken along the direction D1 illustrated in Fig. 7.

As illustrated in Fig. 7, in the present embodiment, the antislip varnish 20b is applied to part of the exposed surface of the wrapping sheet 10, which is exposed when the wrapping sheet 10 individually wraps the absorbent article 1. In the case where the wrapping sheet 10 is folded over in three together with the absorbent article 1 as described above, there are a region in which the number of portions of the wrapping sheet 10 overlapping is two and a region in which the number of portions of the wrapping sheet 10 overlapping is three, as illustrated in Fig. 8. The region in which the number of portions of the wrapping sheet 10 overlapping is two includes a region in which the first region R1 and the third region R3 overlap and a region in which the second region R2 and the third region R3 overlap. In the present embodiment, the antislip varnish 20b is applied to at least part of the region of the exposed surface of the wrapping sheet 10, in which three portions of the wrapping sheet 10, i.e., the first region R1, the second region R2, and the third region R3, overlap.

The individually-wrapped absorbent article 100 becomes the thickest at the region in which three portions of the wrapping sheet 10, i.e., the first region R1, the second region R2, and the third region R3, overlap, and therefore such a region is a region that is most likely to come into contact with another individually-wrapped absorbent article 100 when multiple individually-wrapped absorbent articles 100 are stacked.

Therefore, in the case where multiple individually-wrapped absorbent articles are stacked as illustrated in Fig. 5, the stacked individually-wrapped absorbent articles are less likely to come apart. Therefore, reduction in production efficiency can be avoided when multiple individually-wrapped absorbent articles 100 are stacked and encased in a package. Further, since the antislip varnish 20b is configured to be applied part of the wrapping sheet 10 as described above, increase in cost is minimized.

In the case where the region of the exposed surface of the wrapping sheet 10, in which the three portions, i.e., the first region R1, the second region R2, and the third region R3, overlap, includes a region on which so-called solid printing is performed, the antislip varnish 20b may be applied to the region on which the solid printing is performed. Thus, deterioration of the design, for example, loss of surface glassiness due to the antislip varnish 20b, can be avoided.

### (Third embodiment)

Fig. 9 is a view illustrating a third embodiment of the antislip, and a plan view of the individually-wrapped absorbent article.

As illustrated in Fig. 9, in the present embodiment, antislip is provided through the shape of the fastening tape 30a.

The fastening tape 30a is provided over the first region R1 and the second region R2 across one end 11 of the wrapping sheet 10, similar to the embodiment illustrated in Fig. 1. Moreover, in the present embodiment, the machine MD direction A of the wrapping sheet 10 is the lateral direction D2 of the wrapping sheet 10. Further, the length L1 of the individually-wrapped absorbent article in the longitudinal direction D1 is 90 mm, and the length L2 of the fastening tape 30a in the direction D1 is 38 mm. Specifically, the length L2 of the fastening tape 30a in the direction D1 is 42% relative to the length L1 of the individually-wrapped absorbent article in the longitudinal direction D1. Note that the length L1 of the individually-wrapped absorbent article in the longitudinal direction D1 may be from 40 mm to 170 mm, and the length L2 of the fastening tape 30a in the direction D1 may be from 15 mm to 60 mm.

In the related art, the length of the fastening tape 30a in the direction D1 is 28% relative to the length L1 of the individually-wrapped absorbent article in the longitudinal direction D1.

In the case where multiple individually-wrapped absorbent articles of the related art are stacked as illustrated in Fig. 5, the individually-wrapped absorbent articles are likely to slip in the machine MD direction A. The end side of the fastening tape 30a forms a step with the surface of the wrapping sheet 10. As the length L2 of the fastening tape 30a in the direction orthogonal to the machine MD direction A is increased, the length of the step formed between the fastening tape 30a and the wrapping sheet 10 is increased, so that the individually-wrapped absorbent article is less likely to slip against another individually-wrapped absorbent article when the multiple individually-wrapped absorbent articles are stacked. Use of the fastening tape 30a that fastens one end 11 of the wrapping sheet 10 can make stacked individually-wrapped absorbent articles less slippery and can prevent the stacked individually-wrapped absorbent articles from coming apart without adding an additional structure to the individually-wrapped absorbent article. Note that the length L2 of the fastening tape 30a in the direction D1 is preferably 35% or greater relative to the length L1 of the individually-wrapped absorbent article in the longitudinal direction D1.

Note that, as an antislip structure, an antislip varnish may be applied to a surface of the fastening tape 30a, embossing may be performed on the fastening tape 30a, or the fastening tape 30a itself may be made of a material having a rough surface. Since the fastening tape 30a has the above configuration, the individually-wrapped absorbent article is less likely to slip against another individually-wrapped absorbent article when the multiple individually-wrapped absorbent articles are stacked.

### (Fourth embodiment)

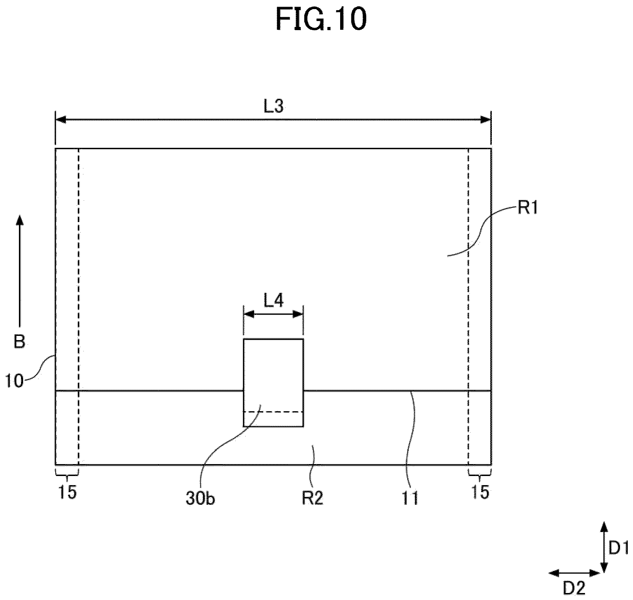

Fig. 10 is a view illustrating a fourth embodiment of the antislip, and is a plan view of the individually-wrapped absorbent article.

As illustrated in Fig. 10, in the present embodiment, the antislip is provided through the shape of the fastening tape 30b.

The fastening tape 30b is provided over the first region R1 and the second region R2 across one end 11 of the wrapping sheet 10, similar to the embodiment illustrated in Fig. 1. Moreover, in the present embodiment, the machine MD direction B of the wrapping sheet 10 is the longitudinal direction D1 of the wrapping sheet 10. Further, the length L3 of the individually-wrapped absorbent article in the lateral direction D2 is 128 mm, and the length L4 of the fastening tape 30a in the direction D1 is 23 mm. Specifically, the length L4 of the fastening tape 30a in the direction D2 is 18% relative to the length L3 of the individually-wrapped absorbent article in the lateral direction D2. Note that the length L3 of the individually-wrapped absorbent article in the lateral direction D2 may be from 50 mm to 200 mm, and the length L4 of the fastening tape 30a in the direction D2 may be from 8 mm to 30 mm.

In the related art, the length of the fastening tape 30a in the direction D2 is 12% relative to the length L3 of the individually-wrapped absorbent article in the lateral direction D2.

In the case where multiple individually-wrapped absorbent articles of the related art are stacked as illustrated in Fig. 5, the individually-wrapped absorbent articles are likely to slip in the machine MD direction B. The end side of the fastening tape 30b forms a step with the surface of the wrapping sheet 10. As the length L4 of the fastening tape 30b in the direction orthogonal to the machine MD direction B is increased, the length of the step between the fastening tape 30b and the wrapping sheet 10 is increased, so that the individually-wrapped absorbent article is less likely to slip in the machine MD direction B when the multiple individually-wrapped absorbent articles are stacked.

Use of the fastening tape 30b that fastens one end 11 of the wrapping sheet 10 can make stacked individually-wrapped absorbent articles less slippery and can prevent the stacked individually-wrapped absorbent articles from coming apart without adding an additional structure to the individually-wrapped absorbent article. Note that the length L4 of the fastening tape 30a in the direction D2 is preferably 15% or greater relative to the length L3 of the individually-wrapped absorbent article in the lateral direction D2.

Note that in the present embodiment, an antislip varnish may be applied to a surface of the fastening tape 30b, embossing may be performed on the fastening tape 30b, or the fastening tape 30b itself may be made of a material having a rough surface, as an antislip structure. Since the fastening tape 30b has the above configuration, the individually-wrapped absorbent article is less likely to slip against another individually-wrapped absorbent article when the multiple individually-wrapped absorbent articles are stacked.

### (Fifth embodiment)

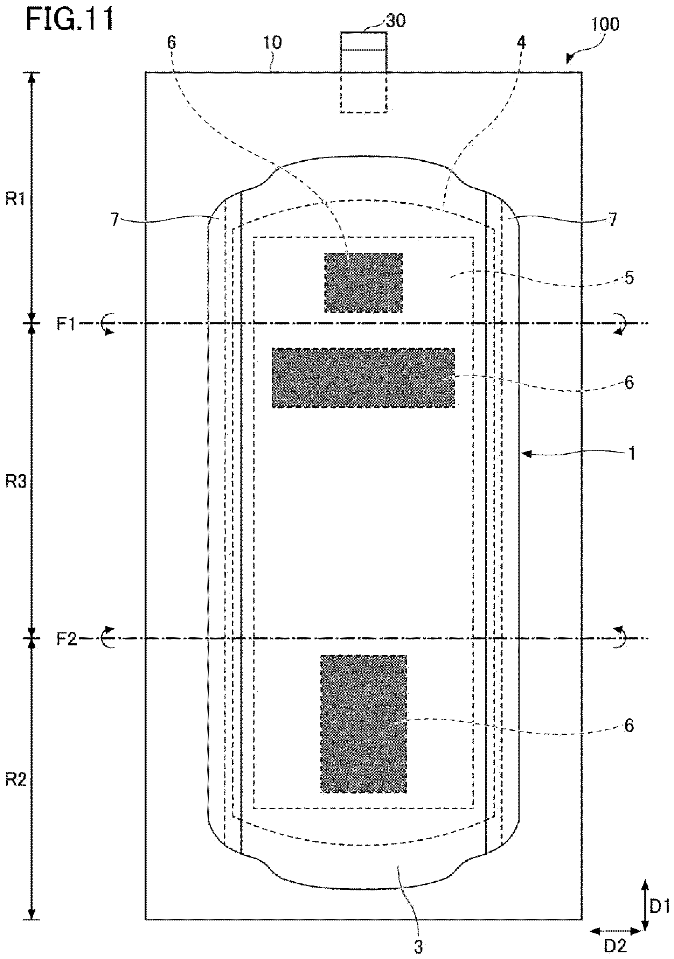

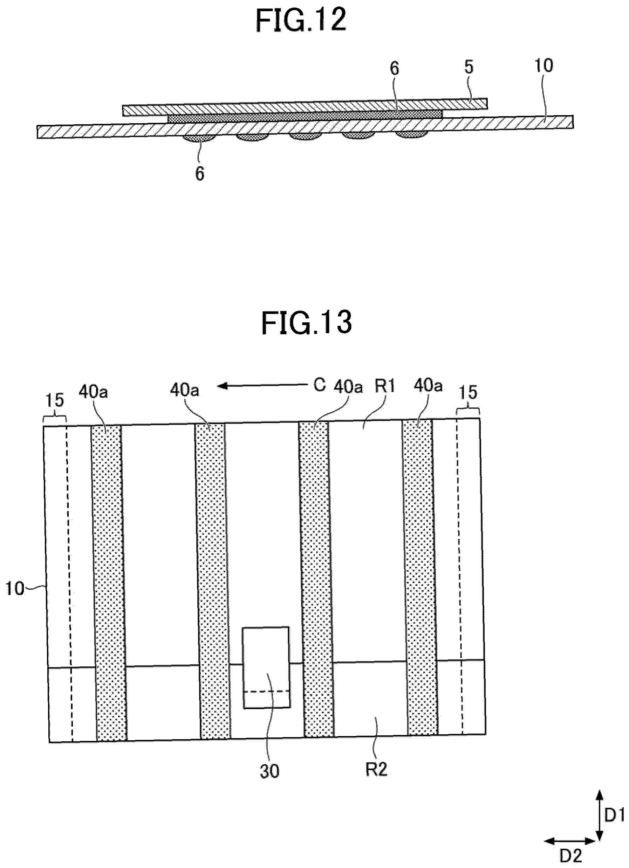

Fig. 11 is a view illustrating a fifth embodiment of the antislip, and is a view illustrating a developed state of the individually-wrapped absorbent article 100 as viewed from the inner surface of the wrapping sheet 10 or the side of the absorbent article 1 facing the skin of a user. Fig. 12 is a cross-sectional view illustrating a stacked state of the release liner 5, the hot-melt adhesive 6, and the wrapping sheet 10, which are illustrated n Fig. 11.

The individually-wrapped absorbent article 100 may include a pseudo-adhesive structure provided to an inner surface of the wrapping sheet 10, i.e., an opposite surface to an exposed surface of the wrapping sheet 10 when the wrapping sheet 10 individually wrap the absorbent article 1, so that the absorbent article 1, which is individually wrapped, does not fall off. The pseudo-adhesive structure is a structure in which the release liner 5 is bonded to the inner surface of the wrapping sheet 10 via a hot-melt adhesive 6 that is one example of an adhesive, and the absorbent article 1 is releasably bonded to the release liner 5, as illustrated in Figs. 11 and 12. Thus, the absorbent article 1 is in the state in which the absorbent article 1 is releasably bonded to the wrapping sheet 10.

In the present embodiment, the hot-melt adhesive 6 seeps out to the exposed surface of the wrapping sheet 10 owing to increase in the amount of the hot-melt adhesive 6 applied. As illustrated in Fig. 12, the hot-melt adhesive 6 may partially seep out to the exposed surface of the wrapping sheet 10, or even if the hot-melt adhesive 6 entirely seeps out, a relief pattern is formed on the exposed surface as the hot-melt adhesive 6 passes through the wrapping sheet 10 in the thickness direction.

Therefore, the hot-melt adhesive 6 seeped to the exposed surface of the wrapping sheet 10 increases a friction force of the exposed surface of the wrapping sheet 10 so that stacked individually-wrapped absorbent articles are less likely to slip against one another. Use of the pseudo-adhesive structure that releasably bonds the absorbent article 1 to the wrapping sheet 10 can make stacked individually-wrapped absorbent articles less slippery without adding an additional structure to the individually-wrapped absorbent article.

### (Sixth embodiment)

Fig. 13 is a view illustrating a sixth embodiment of the antislip, and is a plan view of the individually-wrapped absorbent article.

As illustrated in Fig. 13, in the present embodiment, embossing 40a is provided to multiple portions of the wrapping sheet 10 as the antislip.

In the present embodiment, the machine MD direction C of the wrapping sheet 10 is the lateral direction D2 of the wrapping sheet 10. Moreover, the embossing 40a is provided in linear regions that extend in the longitudinal direction D1 of the wrapping sheet 10 and are parallel to one another. As a pattern formed by the embossing 40a, for example, projected portions or recessed portions, each of which is a circle having a diameter of 5 mm or a rectangle of 5 mm × 5 mm, are regularly arranged.

In the individually-wrapped absorbent article configured in the above manner, the machine MD direction C of the wrapping sheet 10 is the lateral direction D2 of the wrapping sheet 10, and therefore an individually-wrapped absorbent article is likely to slip in the lateral direction of the wrapping sheet 10 when multiple individually-wrapped absorbent articles are stacked.

Since the embossing 40a is provided in the linear regions extending in the longitudinal direction D1 of the wrapping sheet 10, presence and absence of the embossing 40a are repeated in the lateral direction D2 of the wrapping sheet 10 so that an individually-wrapped absorbent article is less likely to slip in the lateral direction D2 of the wrapping sheet 10 when the multiple individually-wrapped absorbent articles are stacked.

### (Seventh embodiment)

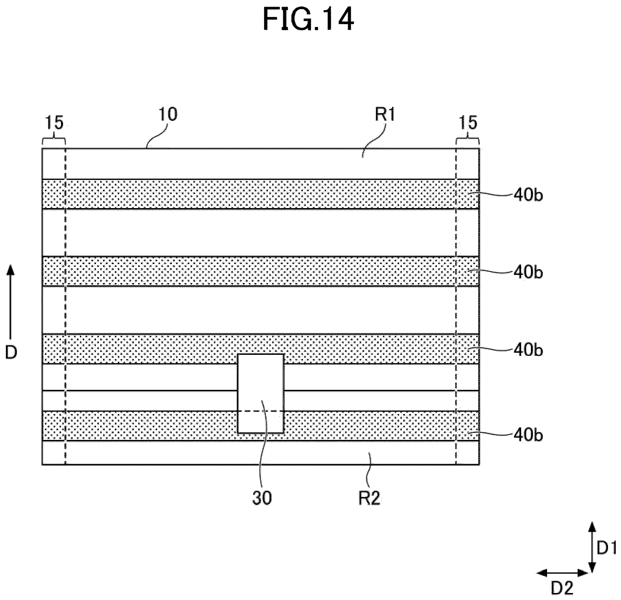

Fig. 14 is a view illustrating a seventh embodiment of the antislip, and is a plan view of the individually-wrapped absorbent article.

As illustrated in Fig. 14, in the present embodiment, embossing 40b is provided to multiple portions of the wrapping sheet 10 as the antislip.

In the present embodiment, the machine MD direction D of the wrapping sheet 10 is the longitudinal direction D1 of the wrapping sheet 10. Moreover, the embossing 40b is provided in linear regions that extend in the lateral direction D2 of the wrapping sheet 10 and are parallel to one another. As a pattern formed by the embossing 40b, for example, projected portions or recessed portions, each of which is a circle having a diameter of 5 mm or a rectangle of 5 mm × 5 mm, are regularly arranged.

In the individually-wrapped absorbent article configured in the above manner, the machine MD direction D of the wrapping sheet 10 is the longitudinal direction D1 of the wrapping sheet 10, and therefore an individually-wrapped absorbent article is likely to slip in the longitudinal direction D1 of the wrapping sheet 10 when multiple individually-wrapped absorbent articles are stacked.

Since the embossing 40b is provided in the linear regions extending the lateral direction D2 of the wrapping sheet 10, presence and absence of the embossing 40b are repeated in the longitudinal direction D1 of the wrapping sheet 10 so that the individually-wrapped absorbent article is less likely to slip in the longitudinal direction D1 of the wrapping sheet 10 when the multiple individually-wrapped absorbent articles are stacked.

### (Eighth embodiment)

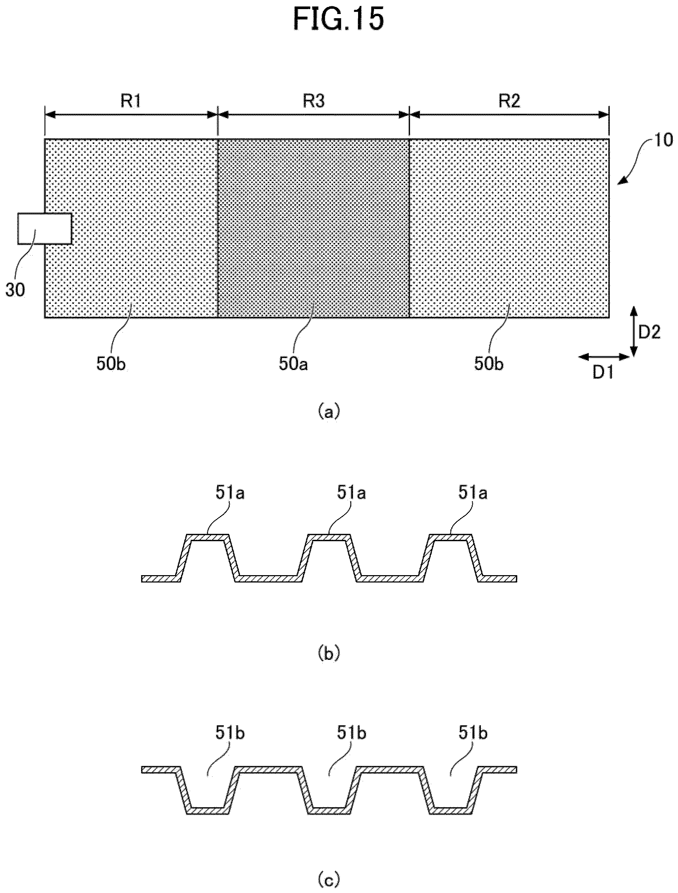

Fig. 15 includes views illustrating an eighth embodiment of the antislip, (a) is a view as viewed from the outer surface of the wrapping sheet 10, (b) is a cross-sectional view illustrating part of an embossed pattern in the third region R3, and (c) is a cross-sectional view illustrating part of an embossed pattern in the first region R1 and the second region R2.

As illustrated in Fig. 15, in the present embodiment, embossing 50a is provided in the third region R3 of the wrapping sheet 10, and embossing 50b is provided in each of the first region R1 and the second region R2. The embossing 50a provided in the third region R3 is configured such that projected portions 51a, which are projected to the outer surface side of the wrapping sheet 10, are regularly arranged as illustrated in Fig. 15(b). Moreover, the embossing 50b provided in each of the first region R1 and the second region R2 is configured such that recessed portions 51b, which are projected towards the inner surface side of the wrapping sheet 10, are regularly arranged as illustrated in Fig. 15(c) .

As described with reference to Figs. 2 and 3, the absorbent article is placed on the inner surface of the wrapping sheet 10 configured in the above manner, and the wrapping sheet 10 is folded so that the surface of the wrapping sheet 10 on which the absorbent article is placed becomes the inner side. Thus, an individually-wrapped absorbent article, in which the absorbent article is individually wrapped with the wrapping sheet 10, is produced.

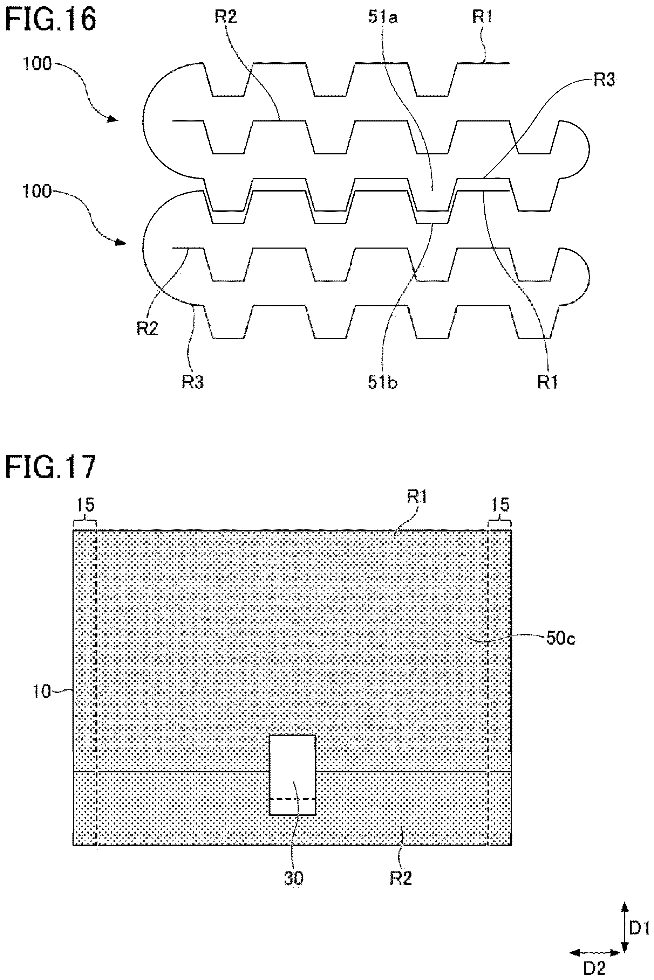

Fig. 16 is a view for explaining a state in which the wrapping sheet 10 illustrated in Fig. 15 is folded, and then the resultant individually-wrapped absorbent articles are stacked.

As illustrated in Fig. 16, when the wrapping sheet 10 illustrated in Fig. 15 is folded and the resultant individually-wrapped absorbent articles are stacked, the first region R1 of the wrapping sheet of one individually-wrapped absorbent article and the third region R3 of the wrapping sheet of another individually-wrapped absorbent article face each other within the stacked individually-wrapped absorbent articles 100. Here, embossing 50b is provided to the first region R1 of the wrapping sheet 10 of the present embodiment, and the embossing 50b is configured such that recessed portions 51b that are recessed toward the inner surface side of the wrapping sheet 10 are regularly arranged. Further, embossing 50a is provided to the third region R3 of the wrapping sheet 10, and the embossing 50a is configured such that projected portions 51a that are projected toward the outer surface side of the wrapping sheet 10 are regularly arranged.

When multiple individually-wrapped absorbent articles 100 are stacked together, the projected portions 51a of the embossing 50a function to respectively form the engaging with the recessed portions 51b of the embossing 50b within the stacked individually-wrapped absorbent articles 100, as illustrated in Fig. 16. Therefore, the individually-wrapped absorbent articles 100 are less likely to slip against one another when the multiple individually-wrapped absorbent articles 100 are stacked.

Note that each projected portion 51a of the embossing 50a or each recessed portion 51b of the embossing 50b may have a size of, for example, a circle having a diameter of 5 mm or a rectangle of 5 mm × 5 mm.

### (Ninth embodiment)

Fig. 17 is a view illustrating a ninth embodiment of the antislip, and is a plan view of the individually-wrapped absorbent article.

As illustrated in Fig. 17, in the present embodiment, embossing is provided to the entire surface of the wrapping sheet 10 as antislip, thereby forming a relief pattern 50c. The relief pattern 50c is formed, for example, by regularly arranging projected portions or recessed portions each in the form of a circle having a diameter of 5 mm or a rectangle of 5 mm × 5 mm.

In the individually-wrapped absorbent article configured in the above manner, the relief pattern 50c is present in the exposed surface of the wrapping sheet 10, and therefore a friction force of the surface is increased, and the individually-wrapped absorbent articles are less likely to slip against one another when the multiple individually-wrapped absorbent articles are stacked.

Note that in the above-described sixth to ninth embodiments, relief patterning serving as antislip is performed on the wrapping sheet by embossing. However, the relief patterning may be provided with application of an ink to the wrapping sheet 10 by dot printing. Moreover, relief patterning may be provided by perforating, such as by cutting out the wrapping sheet 10 from the surface, or perforating the wrapping sheet 10 with a needle.

Further, the antislip described in the first to ninth embodiments may be combined. For example, the surface of the individually-wrapped absorbent article may be made less slippery with the shape of the fastening tape described in the third embodiment or the fourth embodiment, in addition to the application of the antislip varnish to the wrapping sheet as described in the first embodiment or the second embodiment. Moreover, the configuration of the fastening tape described in the third embodiment or the fourth embodiment and the configuration of embossing described in the sixth embodiment or the seventh embodiment may be combined in accordance with the machine MD direction. Further, the surface of the individually-wrapped absorbent article may be made less slippery by providing embossing to the wrapping sheet as described in the sixth to ninth embodiments, in addition to the application of the antislip varnish to the wrapping sheet as described in the first embodiment or the second embodiment.

This international application claims priority based on the Japanese patent application 2023-055945 filed on March 30, 2023, the entire contents of which are incorporated herein by reference.

### DESCRIPTION OF THE REFERENCE NUMERALS

1 absorbent article
3 top sheet
4 absorber
5 release liner
6 hot-melt adhesive
7 side sheet
10 wrapping sheet
11 one end in the longitudinal direction
12 the other end in the longitudinal direction
15 sealed portion
15a pressure-bonded portion
20a, 20b antislip varnish
30, 30a, 30b fastening tape
40a, 40b, 50a, 50b embossing
50c relief pattern
51a projected portion
51b recessed portion
100 individually-wrapped absorbent article
A, B, C, D machine MD direction
D1 longitudinal direction of wrapping sheet
D2 lateral direction of wrapping sheet
F1 first folding line
F2 second folding line
R1 first region
R2 second region
R3 third region

## Claims

1. An individually-wrapped absorbent article, comprising:
a wrapping sheet including paper; and
an absorbent article individually wrapped with the wrapping sheet,
wherein antislip is provided to a surface of the individually-wrapped absorbent article to be in contact with another individually-wrapped absorbent article when multiple individually-wrapped absorbent articles are stacked.

2. The individually-wrapped absorbent article according to claim 1,
wherein an antislip agent is applied to an entire area of an exposed surface of the wrapping sheet, which is exposed when the wrapping sheet individually wraps the absorbent article.

3. The individually-wrapped absorbent article according to claim 1,
wherein within an exposed surface of the wrapping sheet, which is exposed when the wrapping sheet individually wraps the absorbent article, an antislip agent is applied to at least part of a region in which a number of portions of the wrapping sheet overlapping is the largest when the wrapping sheet individually wraps the absorbent article.

4. The individually-wrapped absorbent article according to claim 1, further comprising:
a fastening tape,
wherein the wrapping sheet includes a first region including one end in a longitudinal direction of the wrapping sheet, and a second region including the other end in the longitudinal direction,
the wrapping sheet is folded so that the second region overlaps the first region, and
the fastening tape is provided over the first region and the second region, and
wherein when a machine MD direction of the wrapping sheet is a lateral direction orthogonal to the longitudinal direction, a length of the fastening tape in the longitudinal direction is 35% or greater relative to a length of the wrapping sheet in the longitudinal direction in a state in which the wrapping sheet is folded.

5. The individually-wrapped absorbent article according to claim 1, further comprising:
a fastening tape,
wherein the wrapping sheet includes a first region including one end in a longitudinal direction of the wrapping sheet, and a second region including the other end in the longitudinal direction,
the wrapping sheet is folded so that the second region overlaps the first region,
the fastening tape is provided over the first region and the second region, and
wherein when a machine MD direction of the wrapping sheet is the longitudinal direction, a length of the fastening tape in a lateral direction orthogonal to the longitudinal direction is 15% or greater relative to a length of the wrapping sheet in the lateral direction.

6. The individually-wrapped absorbent article according to claim 4 or 5,
wherein a surface of the fastening tape has an antislip structure.

7. The individually-wrapped absorbent article according to claim 1,
wherein an opposite surface of the wrapping sheet to an exposed surface of the wrapping sheet, which is exposed when the wrapping sheet individually wraps the absorbent article, has a pseudo-adhesive structure that releasably bonds the absorbent article to the wrapping sheet, and
wherein an adhesive constituting the pseudo-adhesive structure seeps through the wrapping sheet to be present on the exposed surface of the wrapping sheet.

8. The individually-wrapped absorbent article according to claim 1,
wherein relief patterning is provided to an exposed surface of the wrapping sheet, which is exposed when the wrapping sheet individually wraps the absorbent article.

9. The individually-wrapped absorbent article according to claim 8,
wherein the relief patterning includes embossing.

10. The individually-wrapped absorbent article according to claim 8,
wherein the relief patterning includes application of an ink by printing.

11. The individually-wrapped absorbent article according to claim 8,
wherein the relief patterning includes perforation of the wrapping sheet.
